# EUROPÄISCHE PATENTANMELDUNG

(11) **EP 2 295 044 A1**
(43) Veröffentlichungstag der Anmeldung: **16.03.2011**
(21) Anmeldenummer: 09011748.2
(22) Anmeldetag: 15.09.2009
(51) Int. Cl.: A61K 9/50, A61K 31/275

(54) **Verkapselung unter Verwendung von wachsartigen Substanzen**

(71) Anmelder: Bayer Technology Services GmbH, 51368 Leverkusen (DE)
(72) Erfinder: Egger, Holger, Dr., 51067 Köln (DE); Storch, Dirk, 51063 Köln (DE)

(57) **Zusammenfassung**

Die vorliegende Erfindung betrifft das Gebiet der Verkapselung von Substanzen. Gegenstand der Erfindung sind Kapseln mit einem hydrophoben, wachsartigen, festen Kern, in den Substanzen wie beispielsweise Wirkstoffe oder Detektionsmittel eingebettet sind, sowie Dispersionen umfassend die erfindungsgemäßen Kapseln. Gegenstand der vorliegenden Erfindung ist ferner die Verwendung erfindungsgemäßer Kapseln zur Verkapselung von Substanzen, ein Verfahren zur Herstellung erfindungsgemäßer Kapseln sowie die Verwendung von hydrophoben, wachsartigen Substanzen zur mechanischen und/oder chemischen Stabilisierung von Kapseln.

## Beschreibung

Die vorliegende Erfindung betrifft das Gebiet der Verkapselung von Substanzen. Gegenstand der Erfindung sind Kapseln mit einem hydrophoben, wachsartigen, festen Kern, in den Substanzen wie beispielsweise Wirkstoffe oder Detektionsmittel eingebettet sind, sowie Dispersionen umfassend die erfindungsgemäßen Kapseln. Gegenstand der vorliegenden Erfindung ist ferner die Verwendung erfindungsgemäßer Kapseln zur Verkapselung von Substanzen, ein Verfahren zur Herstellung erfindungsgemäßer Kapseln sowie die Verwendung von hydrophoben, wachsartigen Substanzen zur mechanischen und/oder chemischen Stabilisierung von Kapseln.

Die Verkapselung von Substanzen wie beispielsweise Wirkstoffen spielt beispielsweise bei der Herstellung von Arzneimitteln eine wichtige Rolle. Durch die Verkapselung werden Wirkstoffe in eine Form gebracht, in der sie besser dem Körper eines Lebewesens verabreicht werden können. Weiterhin kann durch eine Verkapselung eine zeitversetzte Freisetzung eines Wirkstoffs im Körper des Lebewesens erreicht werden.

Damit ist eine Kapsel ein fester Körper, der zur Aufnahme einer Substanz dient. Der homogene Körper einer Matrixkapsel stellt ein Gefüge (eine Matrix) bereit, in den eine Substanz aufgenommen werden kann. Bei Kern-Hülle-Kapseln bildet der homogene Kern die Matrix zur Aufnahme von Substanzen und wird durch eine feste Hülle umschlossen.

Kapseln werden oftmals aufgrund ihrer Größe z.B. als Mikro- oder Nanokapseln bezeichnet, wobei der Übergang zwischen Mikro- und Nanokapseln fließend ist.

Die Hülle von Kern-Hülle-Kapseln wird oftmals durch ein Polymer gebildet. Beispielhaft seien Kern-Hülle-Kapseln mit einer Polyalkyl-Cyanacrylat-Hülle genannt. Sie können beispielsweise in wässriger Phase durch anionische Polymerisation eines Alkylcyanacrylats bei niedrigem pH-Wert in Gegenwart eines sterischen Stabilisators erzeugt werden (S. J. Douglas et al.: Particle Size and Size Distribution of Poly(buryl)-2-cyanoacrylate) Nanoparticles, Journal of Colloid and Interface Science, Vol. 101, No. 1, 1984, Seiten 149-158).

Die Aufnahme von Substanzen in dem Kern der Kapseln kann beispielsweise bereits während der Polymerisation erfolgen, indem die Substanzen vorab in dem Polymerisationsmedium gelöst werden.

T. Pitaksuteepong et al. beschreiben eine Synthese von wirkstoffhaltigen Cyanacrylat-Kapseln durch Polymerisation an den Grenzflächen einer Wasser-in-Öl-Mikroemulsion. Die hydrophilen Wirkstoffe sind in der wässrigen Phase gelöst; durch die Polymerisation an der Grenzfläche Wasser/Öl werden die Wirkstoffe in den wässrigen Tröpfchen eingeschlossen (T. Pitaksuteepong et al.: Factors influencing the entrapment oh hydrophilic compounds in nanocapsules prepared by interfacial polymerisation of water-in-oil microemulsions, European Journal of Pharmaceuticals and Biopharmaceuticals 53 (2002) Seiten 335-342).

Bei hydrophoben Wirkstoffen gelingt eine entsprechende Verkapselung durch Grenzflächenpolymerisation in einer Öl-in-Wasser-Emulsion (siehe z.B. M. Wohlgemuth et al.: Improved preparation and physical studies of polybutylcyanoacrylate nanocapsules, J. Microencapsulation, 2000, Vol. 17, No. 4, Seiten 437-448).

Auf diese Weise hergestellte Kapseln haben bei Raumtemperatur (20°C) eine feste Hülle und einen flüssigen Kern. Sie sind sehr empfindlich gegenüber mechanischer Beanspruchung; die Hülle kann leicht brechen, was ein Auslaufen der Kapseln zur Folge hat.

Für die oben genannte Anwendung von Kapseln zur Aufnahme von beispielsweise Wirkstoffen ist es jedoch erforderlich, dass die Kapseln stabil sind und sich lagern und verarbeiten lassen, ohne dass sie nennenswert geschädigt werden.

Aufgabe der vorliegenden Erfindung ist daher die Bereitstellung von stabilen Kapseln. Die Kapseln sollen lipophile Substanzen aufnehmen können und lagerfähig sowie verarbeitbar sein, ohne dass die Kapseln und/oder die aufgenommenen Substanzen hierbei nennenswert geschädigt werden. Weiterhin sollen die Kapseln im industriellen Maßstab unter wirtschaftlichen Bedingungen herstellbar sein. Eine weitere Aufgabe besteht daher in der Bereitstellung eines wirtschaftlichen und im industriellen Maßstab durchführbaren Verfahrens zur Herstellung stabiler Kapseln.

Überraschend wurde gefunden, dass sich aus einer wässrigen Emulsion hydrophober, wachsartiger Substanzen durch das nachfolgend näher beschriebene Verfahren chemisch und mechanisch stabile Kapseln gewinnen lassen, deren Kern zur Aufnahme von lipophilen Substanzen geeignet ist.

Ein erster Gegenstand der vorliegenden Erfindung ist daher ein Verfahren zur Herstellung von stabilen Kapseln, mindestens umfassend die folgenden Schritte:
(a) Bereitstellen mindestens einer wachsartigen Substanz,
(b) homogenes Vermischen einer zu verkapselnden Substanz mit der mindestens einen wachsartigen Substanz,
(c) Dispergieren der Mischung aus Schritt (b) in einer wässrigen Lösung unter Einsatz mindestens eines Dispergierhilfsmittels,
(d) Abkühlen und Verdünnen der Dispersion aus Schritt (c), um ein Partikelwachstum und/oder eine Agglomeration von Partikeln zu verhindern.

Vorzugsweise werden die Schritte (a) bis (d) in der angegebenen Reihenfolge durchgeführt.

Unter einer wachsartigen Substanz wird eine Substanz verstanden, die hydrophob ist, unter Standardbedingungen fest ist und unterhalb einer Temperatur von 100°C bei Standarddruck in den schmelzeflüssigen Zustand übergeht oder sich unterhalb einer Temperatur von 100°C bei Standarddruck in einer flüssigen, hydrophilen Substanz löst.

Unter Standardbedingungen (englisch NTP = "Normal Temperature and Pressure") werden folgende Bedingungen verstanden:
Standarddruck *p* = 1,01325 bar = 101325 Pa
Standardtemperatur *T* = 298,15 K und dabei ist die Luftdichte ρ = 1,184 kg/m³

Als wachsartige Substanzen eignen sich beispielsweise die unter der Bezeichnung Wachse bekannten Substanzen. Unter einem Wachs wird gemäß der Definition des Römpp Chemie Lexikons (9. Auflage, Georg Thieme Verlag Stuttgart, Band T-Z, Seite 4972) eine Substanz verstanden, die bei 20°C knetbar, fest bis brüchig hart, grob bis feinkristallin, durchscheinend bis opak, jedoch nicht glasartig, bei Temperaturen oberhalb von 40°C unzersetzt schmelzend, schon wenig oberhalb des Schmelzpunkts verhältnismäßig niedrigviskos und nicht fadenziehend ist. Weiterhin zeigt ein Wachs eine stark temperaturabhängige Konsistenz und Löslichkeit und ist unter leichtem Druck polierbar.

Wachse unterscheiden sich von ähnlichen (synthetischen oder natürlichen) Produkten (z.B. Harzen, plastischen Massen, Metallseifen und anderen) hauptsächlich darin, dass sie in der Regel etwa zwischen 50°C und 90°C, in Ausnahmefällen auch bis zu etwa 200°C, in den schmelzflüssigen, niedrigviskosen Zustand übergehen und praktisch frei von aschebildenden Verbindungen sind.

Im Römpp Chemie Lexikon (s.o.) sind auf Seite 4972 in einer Tabelle verschiedene Vertreter der Wachse beispielhaft aufgeführt. Beispiele für Wachse sind Ester von Fettsäuren mit langkettigen (mehr als 24 Kohlenstoffatome), aliphatischen, primären Alkoholen (z.B. Walrat, Bienenwachs, Carnaubawachs), Erdwachse (z.B. Ozokerit, Kenderbal, Neftgil) und Paraffine.

Die Wahl der wachsartigen Substanz richtet sich in erster Linie nach der zu verkapselnden Substanz. Die zu verkapselnde Substanz sollte in der wachsartigen Substanz homogen verteilt werden können. Dies geschieht vorzugsweise durch Lösen der zu verkapselnden Substanz in der verflüssigten wachsartigen Substanz.

Gute Ergebnisse wurden mit Paraffinen, Fettalkoholen, Fettsäuren, Fettsäureestern, Fettsäureethern, Polyethylenwachsen, Montanwachsen, Polyetherwachsen und vor allem Bienenwachs, Cetylalkohol und Luwax E (Montansäureester, BASF SE, Deutschland) als wachsartige Substanzen erzielt. Es ist auch denkbar, mehrere Wachse zu verwenden.

Die zu verkapselnden Substanzen sind vorzugsweise Wirkstoffe oder Detektionsmittel.

Unter einem Wirkstoff wird eine Substanz verstanden, die mit einem biologischen System wechselwirken kann und in oder an dem biologischen System eine Veränderung hervorrufen kann. Beispiele für Wirkstoffe sind Arzneistoffe, Herbizide, Insektizide oder Fungizide.

Unter einem Detektionsmittel wird eine Substanz verstanden, die auf einen äußeren Einfluss eine charakteristische Antwort zeigt. Vorzugsweise reichern sich die Detektionsmittel in einem Organismus an einer bestimmten Stelle an und sind hier durch chemische oder physikalische Methoden nachweisbar. Beispielhaft seien Fluoreszenzmarker angeführt, die bei Bestrahlung mit elektromagnetischer Strahlung bestimmter Wellenlängen ihrerseits elektromagnetische Strahlung mit einem charakteristischen Wellenlängenmuster aussenden. Ein weiteres Beispiel eines Detektionsmittels sind Radionuklide.

Die Konzentration der verkapselten Substanz ist abhängig von der verwendeten Substanz und dem Anwendungszweck. Werden als Substanzen Wirkstoffe wie beispielsweise Deltamethrin, Flumethrin, Clotrimazol, Bifonazol und/oder Transfluthrin verwendet, liegt die Konzentration bezogen auf das Gewicht der Kapsel im Bereich von 0,01 bis 50 Gew.-%, bevorzugt im Bereich von 1 bis 30 Gew.-%, besonders bevorzugt im Bereich von 5 bis 15 Gew.-%.

Es ist denkbar, die wachsartige Substanz mit hydrophoben, unter Standardbedingungen flüssigen Substanzen zu mischen, bevor eine Dispergierung in einer wässrigen Lösung erfolgt. Es kann zum Beispiel vorteilhaft sein, der wachsartigen Substanz ein Öl zuzusetzen oder die zu verkapselnde Substanz zunächst in einem Öl zu lösen, bevor das Öl-Gemisch mit der wachsartigen Substanz vermischt wird, um eine homogenere Verteilung der zu verkapselnden Substanz in den Kapseln zu erzielen.

Eventuell kann es hilfreich sein, geringe Mengen an Lösemittel wie beispielsweise Ethanol zuzugeben, um eine homogene Verteilung des Gemisches zu erreichen.

Ebenso kann durch Zusatz einer flüssigen hydrophoben Substanz erreicht werden, dass bei einer Temperatur unterhalb von 100°C eine flüssige Mischung vorliegt, die in einer wässrigen Lösung dispergiert werden kann, auch wenn die wachsartige Substanz unter Standarddruck erst oberhalb von 100°C schmilzt.

In einer bevorzugten Ausführungsform erfolgt der Schritt (b) des erfindungsgemäßen Verfahrens daher unter Zugabe einer hydrophoben, unter Standardbedingungen flüssigen Substanz.

Die Wahl der hydrophoben, unter Standardbedingungen flüssigen Substanz richtet sich nach der zu verkapselnden Substanz und der ausgewählten wachsartigen Substanz. Alle verwendeten Substanzen sollten homogen miteinander vermischbar sein. Ferner sollte die Mischung unter Standardbedingungen fest sein.

Als hydrophobe, unter Standardbedingungen flüssige Substanzen eignen sich beispielsweise Öle. Gemäß der Definition des Römpp Chemie Lexikons (9. Auflage, Georg Thieme Verlag Stuttgart, Band TM-Pk, Seite 3094) wird unter einem Öl eine organische Substanz verstanden, die wasserunlöslich und bei Raumtemperatur flüssig ist. Beispiele sind Mineralöle, die aus Erdöl gewonnen werden, synthetische Öle wie z.B. Silikonöl, Triglyceride mittlerer gesättigter oder ungesättigter Fettsäuren (pflanzliche und tierische fette Öle).

Gute Ergebnisse wurden mit flüssigen Fettsäureestern wie beispielsweise Miglyol 812 (Caesar & Loretz GmbH, Deutschland), einem Gemisch aus Decanoyl- und Octanoylglyceriden, sowie aliphatischen und aromatischen Kohlenwasserstoffen und Kohlenwasserstoffgemischen wie beispielsweise Solvesso-200 (CAS-Nr. 64742-94-5, F.B. Silbermann GmbH& Co KG) erzielt.

Es ist auch denkbar, dass eine größere Menge (bezogen auf das Gesamtgewicht der Kapseln) an hydrophober flüssiger Substanz eingesetzt wird als an wachsartiger Substanz.

Bezogen auf die Summe der zur Ausbildung der Kapseln eingesetzten Substanzen liegt die Konzentration der eingesetzten wachsartigen Substanz im Bereich von 0,01 bis 100 Gew.-%, bevorzugt im Bereich von 1 bis 30 Gew.-%, besonders bevorzugt im Bereich von 5 bis 15 Gew.-%.

In Schritt (c) wird die homogene hydrophobe Mischung unter Verwendung eines oder mehrerer Dispergierhilfsmittel bei einer Temperatur oberhalb des Erstarrungspunktes der Mischung in einer wässrigen Lösung dispergiert.

Als Dispergierhilfsmittel werden üblicherweise Tenside eingesetzt. Es können ionische (kationische, anionische, zwitterionische) und nichtionische Tenside als Dispergierhilfsmittel verwendet werden. Auch amphiphile Blockcopolymere lassen sich einsetzen.

Beispiele für geeignete Dispergierhilfsmittel sind Alkoxylate, Alkylolamide, Ester, Aminoxide, Alkylpolyglukoside, Alkylphenole, Arylalkylphenole, wasserlösliche Homopolymere, statistische Copolymere, Blockcopolymere, Pfropfpolymere, Polyethylenoxide, Polyvinylalkohole, Copolymere aus Polyvinylalkoholen und Polyvinylacetaten, Polyvinylpyrrolidone, Cellulose, Stärke, Gelatine, Gelatinederivate, Aminosäurepolymere, Polylysin, Polyasparaginsäure, Poly(meth)acrylate, Polyethylensulfonate, Polystyrolsulfonate, Kondensationsprodukte von aromatischen Sulfonsäuren mit Formaldehyd, Naphthalinsulfonate, Ligninsulfonate, Copolymerisate acrylischer Monomere, Polyethylenimine, Polyvinylamine, Polyallylamine, Poly(2-vinylpyridine) und/oder Polydiallyldimethylammoniumchlorid. Auch Mischungen von verschiedenen Dispergierhilfsmitteln können verwendet werden.

Vorzugsweise werden Polyoxyethylen-polyoxypropylen-Blockcopolymere wie beispielsweise Synperonic F68 (Handelsbezeichnung von ICI, Großbritannien) und/oder ein Copolymer mit pigmentaffinen Gruppen wie beispielsweise Disperbyk-192 (BYK-Chemie GmbH, Deutschland) eingesetzt.

Das Dispergierhilfsmittel wird vorzugsweise in der wässrigen Phase gelöst, bevor die hydrophobe Mischung hinzugefügt wird. Die Konzentration des Dispergierhilfsmittels in der wässrigen Phase liegt im Bereich von 0,1 - 50 Gew.-%, bevorzugt im Bereich von 1 - 30 Gew.-%.

Vorzugsweise wird die flüssige hydrophobe Mischung unter Rühren zur wässrigen Phase gegeben. Es ist denkbar, die Dispergierung durch den Einsatz von Ultra-Turrax oder Ultraschall zu unterstützen. Der Einsatz von hohen Energieeinträgen durch Ultra-Turrax oder Ultraschall ist jedoch nicht erforderlich und für einen Prozess im industriellen Maßstab auch eher unerwünscht. Durch Erhöhung der Konzentration an Dispergierhilfsmittel gelingt die Dispergierung auch unter üblichem Rühren.

In Schritt (d) wird die Emulsion verdünnt und auf eine Temperatur zwischen 0° und 30°C abgekühlt, um eine Koaleszenz der hydrophoben Tröpfchen und ein Partikelwachstum zu vermeiden. Durch Routineversuche lässt sich im Einzelfall bestimmen, auf welche Temperatur die Emulsion abgekühlt werden muss und welche Verdünnung erforderlich ist, um eine Koaleszenz der hydrophoben Tröpfchen und ein Partikelwachstum zu vermeiden.

Die Verdünnung erfolgt vorzugsweise im Verhältnis Wasser:Emulsion von 1:1 bis 10:1. Die Abkühlung erfolgt bevorzugt auf eine Temperatur unterhalb des Erstarrungspunkts der Mischung, welche die Partikel bildet. Die Abkühlung und/oder Verdünnung erfolgt vorzugsweise schnell, wobei unter "schnell" verstanden wird, dass die Verdünnung und/oder Abkühlung ohne vermeidbare Verzögerung in der kürzesten Zeit erfolgt, die technisch und unter wirtschaftlichen Bedingungen sowie im Hinblick auf die erforderlichen Sicherheitsbestimmungen realisierbar ist.

Es ist z.B. denkbar, die erhitzte Emulsion unter Rühren in Wasser mit einer Temperatur zwischen 0° und 30°C zu geben, um eine Verdünnung und Abkühlung zu erreichen.

Das Ergebnis ist eine wässrige Dispersion von festen, meist sphärischen Partikeln mit einem maximalen Durchmesser von weniger als 1 µm, vorzugsweise von weniger als 0,5 µm, besonders bevorzugt von weniger als 0,25 µm. Der über eine Vielzahl von Partikel arithmetisch gemittelte maximale Durchmesser liegt im Bereich von 20 - 200 nm, vorzugsweise im Bereich von 50 - 120 nm. Die Bestimmung der Durchmesser kann beispielsweise anhand elektronenmikroskopischer Aufnahmen erfolgen.

Die Partikel bilden eine Matrix (Kapsel), in der die zu verkapselnden Substanzen eingebettet sind. Die feste wachsartige Matrix ist mechanisch stabil und schützt die eingebetteten Substanzen vor chemischer und/oder physikalischer Schädigung, beispielsweise vor Oxidation oder Schädigung durch UV-Strahlung. Es ist überraschend, dass die hydrophoben Partikel mit einer wachsartigen Substanz auch ohne eine Hülle, wie sie beispielsweise bei den aus dem Stand der Technik bekannten Kern-Hülle-Kapseln mit einem flüssigen Kern vorliegen, eine hohe Stabilität aufweisen und lagerstabil sind.

Die nach dem erfindungsgemäßen Verfahren gewonnenen Kapseln sind ebenfalls Gegenstand der vorliegenden Erfindung. Die erfindungsgemäßen Kapseln sind **dadurch gekennzeichnet, dass** sie mindestens eine wachsartige Substanz und eine zu verkapselnde Substanz enthalten und einen maximalen Durchmesser von weniger als 1 µm, vorzugsweise von weniger als 0,5 µm, besonders bevorzugt von weniger als 0,25 µm aufweisen. In einer bevorzugten Ausführungsform umfassen die erfindungsgemäßen Kapseln weiterhin eine oder mehrere hydrophobe, unter Standardbedingungen flüssige Substanz, die mit der wachsartigen Substanz homogen vermischt ist.

Bevorzugt werden die erfindungsgemäßen Kapseln in wässriger Dispersion aufbewahrt. Ein weiterer Gegenstand der vorliegenden Erfindung ist daher eine wässrige Dispersion umfassend erfindungsgemäße wachshaltige Kapseln sowie ein oder mehrere Dispergierhilfsmittel.

Es ist denkbar, den pH-Wert der Dispersion gezielt einzustellen, um die Lagerstabilität zu erhöhen. Bevorzugt wird die Dispersionen auf einen pH-Wert ≥ 7, besonders bevorzugt auf pH 9 - 10 eingestellt. Dies kann beispielsweise durch Verwendung geeigneter Puffer erreicht werden.

Es ist denkbar, das Wasser durch eine andere flüssige Phase zu ersetzen, beispielsweise durch Alkohole, wie Ethanol oder Methanol. Ebenso ist es denkbar, das Wasser durch gängige Verfahren wie beispielsweise Sprühtrocknen zu entfernen und die Kapseln trocken zu lagern.

Es ist denkbar, die erfindungsgemäßen Kapseln mit einer Hülle zu umgeben. Eine Hülle ist, wie oben dargestellt, im Hinblick auf eine mechanische Stabilität der Kapseln nicht zwingend erforderlich. Trotzdem kann eine zusätzliche Hülle die Lagerstabilität positiv beeinflussen, in dem mögliche Agglomeration der Partikel weiter reduziert wird. Eine Hülle kann außerdem vorteilhaft sein, um z.B. eine Diffusionsbarriere für den eingeschlossenen Wirkstoff darzustellen und somit ein zeitliches Freisetzungsprofil zu erreichen oder um eine Barriere für Stoffe (z.B. Wasser oder Sauerstoff) zu bilden, die zu einer Schädigung der verkapselten Substanz führen könnten. In einer bevorzugten Ausführungsform weisen die erfindungsgemäßen Kapseln eine Hülle auf.

Bevorzugt werden die erfindungsgemäßen Kapseln mit einer Polymerhülle versehen, besonders bevorzugt mit einer Hülle aus Polyalkylcyanacrylat. Der Alkylrest des Polyalkylcyanacrylats ist vorzugsweise eine C₁-C₈-Kette. Besonders bevorzugt besteht die Hülle aus Polymethylcyanacrylat, Polyethylcyanacrylat, Polypropylcyanacrylat, Polybutylcyanacrylat oder einem Mischpolymer der genannten Polyalkylcyanacrylate.

In einer bevorzugten Ausführungsform umfasst das erfindungsgemäße Verfahren zur Herstellung von stabilen Kapseln somit den weiteren Schritt
(e) Umhüllen der Kapseln mit einer Polymerhülle.

Schritt (e) wird im Anschluss an Schritt (c) oder (d) ausgeführt. Vorzugsweise erfolgt die Ausführung des Schritts (e) nach dem Schritt (d). Es ist denkbar, den Schritt (e) auch noch nach einer Lagerung der nicht-umhüllten Kapseln über einen Zeitraum von Stunden, Tagen oder Monaten durchzuführen.

Vorzugsweise wird die Polymerhülle durch Polymerisation in der wässrigen Dispersion der Kapseln an den Phasengrenzflächen wässrige Lösung/Kapseln aufgebaut.

Hierzu lassen sich solche Monomere einsetzen, die bevorzugt an der Phasengrenzfläche polymerisieren. Es können auch Monomergemische eingesetzt werden. Bevorzugt werden n-Alkylcyanacrylate eingesetzt.

Vorzugsweise werden die Monomere in einem geeigneten Lösemittel in die wässrige Phase gegeben. Als Lösemittel für Alkylcyanacrylate eignen sich beispielsweise Methanol, Ethanol, Aceton, Dichlormethan, Chloroform, Isopropanol, Tetrahyfrofuran. Vorzugsweise wird Aceton eingesetzt.

Das Lösemittel wird vorzugsweise "angesäuert", d.h. z.B. mit einer Menge von 0,01 bis 10 Gew.-% Salzsäure versetzt, um eine vorzeitige Reaktion des Monomers zu unterbinden.

Die Konzentration des Monomers in dem Lösemittel liegt üblicherweise im Bereich von 0,1 bis 10 Gew.-%.

Die Polymerisation kann bei Raumtemperatur (10-30°C) erfolgen. Ebenso kann es sinnvoll sein, die Reaktion bei niedrigerer oder höherer Temperatur durchzuführen. Eine niedrigere Temperatur kann z.B. sinnvoll sein, um Nebenreaktionen zu unterbinden. Eine höhere Temperatur kann z.B. sinnvoll sein, um die Reaktion zu beschleunigen.

Je nach verwendeten Monomeren kann es sinnvoll sein, zur Reaktionsbeschleunigung und/oder zur Aktivierung einen Katalysator einzusetzen. Zur Reaktionsbeschleunigung tragen auch beispielsweise funktionelle Gruppen wie Polyether oder Hydroxylfunktionen bei.

Auch der Einsatz von elektromagnetischer Strahlung zur Initiierung und/oder Beschleunigung der Polymerisation kann je nach verwendetem Monomer sinnvoll sein.

Die erfindungsgemäßen umhüllten Kapseln zeigen gegenüber den aus dem Stand der Technik bekannten Kern-Hülle-Kapseln mit einem flüssigen Kern eine deutlich höhere Stabilität. Diese wird durch die wachsartige Substanz im Kern verursacht, die den Kern unter Standardbedingungen fest werden lässt. Gleichzeitig sind die verkapselten Substanzen durch den festen Kern gegenüber chemischen oder physikalischen Schädigungen (Oxidation, UV-Strahlung) geschützt.

Daher ist die Verwendung von wachsartigen Substanzen zur Erhöhung der mechanischen und/oder chemischen Stabilität von Kapseln, insbesondere von Kern-Hülle-Kapseln ein weiterer Gegenstand der vorliegenden Erfindung.

Auch die Verwendung erfindungsgemäßer Kapseln zur Verkapselung von Substanzen wie Wirkstoffen oder Detektionsmitteln ist Gegenstand der vorliegenden Erfindung.

Die Erfindung wird nachstehend anhand von Beispielen näher erläutert, ohne sie jedoch hierauf zu beschränken.

### Beispiel 1: Kapseln enthaltend Deltamethrin ohne wachsartige Substanz (Vergleichsbeispiel)

100 g Solvesso 200, in denen 10 wt% Deltamethrin (3-(2,2-Dibromethenyl-)-2,2-dimethylcyclopropan)) gelöst wurden, und 300 g wässrige Phase, in der 1 wt% Synperonic F68 gelöst und mit Puffer auf pH 7 gestellt wurden, wurden zunächst mit einem Utraturrax zu einer Öl-in-Wasser-Emulsion emulgiert. Anschließend wurde der Ultraturrax durch einen Rührer mit 1000 U/min ersetzt. Unter Rühren wurden 22.5 g einer Mischung aus Ethanol und 1 N HCl (Verhältnis 1000:1) und 1.8 g Ethylcyanacrylat zugetropft und für eine Stunde nachgerührt.

### Beispiel 2: Kapseln enthaltend Deltamethrin mit Bienenwachs

400 g wässrige Phase, in der 0.5 wt% Synperonic F68 gelöst und mit Puffer auf pH 7 gestellt wurden, wurden in einem heißen Wasserbad auf ca. 60 °C erwärmt. Anschließend wurden darin 22.5 g Solvesso 200, in denen 10 wt% Deltamethrin und 10 wt% Bienenwachs ebenfalls im heißen Wasserbad gelöst wurden, mit einem Utraturrax zu einer Öl-in-Wasser-Emulsion emulgiert. Anschließend wurde der Ultraturrax durch einen Rührer mit 300 U/min ersetzt. Unter Rühren wurden 22.5 g einer Mischung aus Ethanol und 1 N HCl (Verhältnis 1000:1) und 1.8 g Ethylcyanacrylat zugetropft und für dreißig Minuten nachgerührt. Anschließend wurde die Mischung mit einem kalten Wasserbad auf Raumtemperatur abgekühlt.

### Beispiel 3: Kapseln enthaltend Flumethrin mit Bienenwachs

200 g wässrige Phase, in der 2 wt% Disperbyk 192 gelöst und mit Puffer auf pH 7 gestellt wurden, wurden in einem heißen Wasserbad auf ca. 60 - 70 °C erwärmt. Anschließend wurden darin 11.25 g Miglyol 812, in denen 20 wt% Flumethrin und 10 wt% Bienenwachs ebenfalls im heißen Wasserbad gelöst wurden, mit einem Utraturrax zu einer Öl-in-Wasser-Emulsion emulgiert und dann schnell auf Raumtemperatur abgekühlt. Anschließend wurde der Ultraturrax durch einen Rührer mit 300 U/min ersetzt. Unter Rühren wurden 11.25 g einer Mischung aus Ethanol und 1 N HCl (Verhältnis 1000:1) und 1.8 g Ethylcyanacrylat zugetropft und für eine Stunde nachgerührt.

Die Partikelgröße wurde mittels Transmissionselektronenmikroskopie (TEM) ermittelt. Die Durchmesser der Kapseln liegt im Bereich von 80 - 150 nm.

Anschließend wurde die Dispersion bei Raumtemperatur für 6 Monate und bei 40 °C für 4 Wochen gelagert. In diesem Zeitraum blieb die Probe stabil, d.h. es wurde keine Agglomeration der Partikel, keine Veränderung der Partikelgröße und kein Austritt von Wirkstoff oder Kernmatrix beobachtet.

### Beispiel 4: Kapseln enthaltend Flumethrin mit Bienenwachs

100 g wässrige Phase, in der 30 wt% Disperbyk 192 gelöst und mit Puffer auf pH 10 gestellt wurden, wurden in einem heißen Wasserbad auf ca. 60 - 80 °C erwärmt. Anschließend wurden darin 11.2 g Miglyol 812, in denen 10 wt% Flumethrin (Alpha-Cyano-(4-fluoro-3-phenoxy)-benzyl-3-[2-chloro-2-(4-chlorophenyl)-ethenyl]-2,2-dimethyl-cyclopropancarboxylat) und 10 wt% Bienenwachs ebenfalls im heißen Wasserbad gelöst wurden, unter Rühren emulgiert. 10 g dieser Mischung wurde mit der zehnfachen Menge (100 g) auf pH 9 gestelltem Wasser verdünnt und unter starkem Rühren wurden 10 g einer Mischung aus Aceton und 10 gewichtsprozentiger Salzsäure (Verhältnis 500:1) und 0.4 g Ethylcyanacrylat zugetropft. Anschließend wurde bei 40 °C überschüssiges Aceton am Rotationsverdampfer entfernt. Schließlich wurde zur Stabilisierung Puffer 10 borate (Fisher Scientific) zugegeben.

Die Partikelgröße wurde mittels TEM in Negativkontrastierung ermittelt. Der Durchmesser der Kapseln liegt im Bereich von 50 - 120 nm.

### Beispiel 5: Kapseln enthaltend Deltamethrin mit Bienenwachs

100 g wässrige Phase, in der 30 wt% Disperbyk 192 gelöst und mit Puffer auf pH 10 gestellt wurden, wurden in einem heißen Wasserbad auf ca. 60 - 80 °C erwärmt. Anschließend wurden darin 11.2 g Miglyol 812, in denen 10 wt% Deltamethrin und 10 wt% Bienenwachs ebenfalls im heißen Wasserbad gelöst wurden, unter Rühren emulgiert.

10 g dieser Mischung wurde mit der zehnfachen Menge (100 g) auf pH 9 gestelltem Wasser verdünnt und unter starkem Rühren wurden 20 g einer Mischung aus Aceton und 10 gewichtsprozentiger Salzsäure (Verhältnis 500:1) und 0.8 g Ethylcyanacrylat zugetropft. Anschließend wurde bei 40 °C überschüssiges Aceton am Rotationsverdampfer entfernt. Schließlich wurde zur Stabilisierung Puffer 10 borate (Fisher Scientific) zugegeben.

Anschließend wurde die Dispersion (gepuffert und ungepuffert) bei Raumtemperatur und bei 40 °C für 2 Wochen gelagert. In diesem Zeitraum blieb die gepufferte Probe (bei RT und 40 °C) sowie die ungepufferte (bei RT) stabil, d.h. es wurde keine Agglomeration der Partikel, keine Veränderung der Partikelgröße und kein Austritt von Wirkstoff oder Kernmatrix beobachtet.

Die ungepufferte Probe gelagert bei 40 °C zeigte dagegen beginnende Sedimentation der Partikel.

### Beispiel 6: Kapseln enthaltend Deltamethrin mit Cetylalhohol

100 g wässrige Phase, in der 30 wt% Disperbyk 192 gelöst wurden, wurden in einem heißen Wasserbad auf ca. 80 - 90 °C erwärmt. Anschließend wurden darin 14,9 g Miglyol 812, in denen 10 wt% Deltamethrin und 10 wt% Cetylalkohol ebenfalls im heißen Wasserbad gelöst wurden, unter Rühren emulgiert.

12,8 g dieser Mischung wurde mit 100g Wasser verdünnt und unter starkem Rühren wurden 20 g einer Mischung aus Aceton und 10 gewichtsprozentiger Salzsäure (Verhältnis 500:1) und 0.2 g Ethylcyanacrylat zugetropft. Anschließend wurde bei 40 °C überschüssiges Aceton am Rotationsverdampfer entfernt.

Anschließend wurde die Dispersion bei Raumtemperatur, bei 40 °C und bei 60 °C für 4 Tage gelagert. In diesem Zeitraum blieben die Proben Raumtemperatur und bei 40 °C stabil, d.h. es wurde keine Agglomeration der Partikel, keine Veränderung der Partikelgröße und kein Austritt von Wirkstoff oder Kernmatrix beobachtet. Bei 60 °C waren geringe Ablagerungen am Rand zu erkennen; der überwiegende Teil der Dispersion blieb aber ebenfalls stabil.

## Patentansprüche

1. Verfahren zur Herstellung von stabilen Kapseln, mindestens umfassend die folgenden Schritte:
(a) Bereitstellen mindestens einer wachsartigen Substanz,
(b) homogenes Vermischen einer zu verkapselnden Substanz mit der mindestens einen wachsartigen Substanz,
(c) Dispergieren der Mischung aus Schritt (b) in einer wässrigen Lösung unter Einsatz mindestens eines Dispergierhilfsmittels,
(d) Abkühlen und Verdünnen der Dispersion aus Schritt (c).

2. Verfahren nach Anspruch 1, **dadurch gekennzeichnet, dass** die wachsartige Substanz ausgewählt wird aus der Reihe: Paraffin, Fettalkohol, Fettsäure, Fettsäureester, Fettsäureether, Polyethylenwachs, Montanwachs, Polyetherwachs, Bienenwachs, Cetylalkohol und/oder Montansäureester.

3. Verfahren nach einem der Ansprüche 1 oder 2, **dadurch gekennzeichnet, dass** als zu verkapselnde Substanz ein Wirkstoff oder ein Detektionsmittel eingesetzt wird.

4. Verfahren nach einem der Ansprüche 1 bis 3, **dadurch gekennzeichnet, dass** Schritt (b) unter Zugabe mindestens einer hydrophoben, unter Standardbedingungen flüssigen Substanz erfolgt.

5. Verfahren nach einem der Ansprüche 1 bis 4, **dadurch gekennzeichnet, dass** die hydrophobe, unter Standardbedingungen flüssige Substanz ein Fettsäureester oder Fettsäureestergemisch oder ein aliphatisches oder aromatisches Kohlenwasserstoffgemisch ist.

6. Verfahren nach einem der Ansprüche 1 bis 5, **dadurch gekennzeichnet, dass** als Dispergierhilfsmittel ein Polyoxyethylen-polyoxypropylen-Blockcopolymer eingesetzt wird.

7. Verfahren nach einem der Ansprüche 1 bis 6, umfassend den weiteren Schritt (e) Umhüllen der Kapseln mit einer Polymerhülle.

8. Verfahren nach einem der Ansprüche 1 bis 7, **dadurch gekennzeichnet, dass** die Polymerhülle durch Polymerisation in der wässrigen Dispersion der Kapseln an den Phasengrenzflächen wässrige Lösung/Kapseln aufgebaut wird.

9. Kapseln umfassend mindestens eine wachsartige Substanz und eine zu verkapselnde Substanz, **dadurch gekennzeichnet, dass** sie einen maximalen Durchmesser von weniger als 1 µm, vorzugsweise von weniger als 0,5 µm, besonders bevorzugt von weniger als 0,25 µm aufweisen.

10. Kapseln nach Anspruch 9, weiterhin umfassend eine oder mehrere hydrophobe, unter Standardbedingungen flüssige Substanzen, die mit der wachsartigen Substanz homogen vermischt sind.

11. Kapseln nach einem der Ansprüche 9 oder 10, weiterhin umfassend eine Polymerhülle.

12. Kapseln nach einem Anspruch 11, **dadurch gekennzeichnet, dass** die Polymerhülle aus Polyalkylcyanacrylat besteht.

13. Wässrige Dispersion, mindestens umfassend Kapseln gemäß einem der Ansprüche 9 bis 12 und mindestens ein Dispergierhilfsmittel.

14. Verwendung von wachsartigen Substanzen zur Erhöhung der mechanischen und/oder chemischen Stabilität von Kapseln und/oder verkapselten Substanzen.

15. Verwendung von Kapseln nach einem der Ansprüche 9 bis 12 zur Verkapselung einer oder mehrerer Wirkstoffe oder eines oder mehrerer Detektionsmittel.
